(19) Europäisches Patentamt
European Patent Office
Office européen des brevets

(11) EP 0 850 637 B2

(12) NOUVEAU FASCICULE DE BREVET EUROPEEN
Après la procédure d'opposition

(45) Date de publication et mention de la décision concernant l'opposition:
**10.11.2010 Bulletin 2010/45**

(45) Mention de la délivrance du brevet:
**02.06.1999 Bulletin 1999/22**

(21) Numéro de dépôt: **97402848.2**

(22) Date de dépôt: **26.11.1997**

(51) Int Cl.:
***A61K 8/41*** *(2006.01)* ***A61K 8/49*** *(2006.01)*
***A61Q 5/10*** *(2006.01)*

(54) **Composition de teinture d'oxydation des fibres kératiniques et procédé de teinture mettant en oeuvre cette composition**

Zusammensetzung zur Oxidationsfärbung von keratinischen Fasern und Färbeverfahren mit dieser Zusammensetzung

Composition for the oxidative dyeing of keratinic fibres and dyeing process using this composition

(84) Etats contractants désignés:
**AT BE CH DE DK ES FI FR GB GR IE IT LI LU MC NL PT SE**

(30) Priorité: **23.12.1996 FR 9615891**

(43) Date de publication de la demande:
**01.07.1998 Bulletin 1998/27**

(73) Titulaire: **L'Oréal**
**75008 Paris (FR)**

(72) Inventeurs:
- **Rondeau, Christine**
**18500 Sartrouville (FR)**
- **Cotteret, Jean**
**78480 Verneuil Sur Seine (FR)**
- **De La Mettrie, Roland**
**78110 Le Vesinet (FR)**

(74) Mandataire: **Dossmann, Gérard**
**Casalonga & Partners**
**Bayerstrasse 71-73**
**80335 München (DE)**

(56) Documents cités:

| | |
|---|---|
| **EP-A- 0 739 622** | **WO-A1-95/01772** |
| **WO-A1-95/15144** | **WO-A1-97/39727** |
| **DE-A- 2 543 100** | **US-A- 4 025 301** |



Printed by Jouve, 75001 PARIS (FR)

## Description

La présente invention a pour objet une composition prête à l'emploi pour la teinture d'oxydation des fibres kératiniques, en particulier des fibres kératiniques humaines telles que les cheveux, comprenant, dans un milieu approprié pour la teinture, au moins une base d'oxydation choisie parmi les paraphénylènediamines et les bis-phénylalkylènediamines, en association avec au moins un coupleur choisi parmi les méta-diphénols, au moins un colorant direct cationique sélectionné et au moins un agent oxydant, ainsi que le procédé de teinture mettant en oeuvre cette composition. Elle concerne également un kit de coloration pour la préparation d'une telle composition prête à l'emploi.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

Il est également connu que pour faire encore varier les nuances obtenues et leur donner des reflets, on peut utiliser, en association avec les précurseurs de colorants d'oxydation et les coupleurs, des colorants directs, c'est à dire des substances colorées qui apportent une coloration en l'absence d'agent oxydant.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants directs appartiennent pour leur très grande majorité à la famille des composés nitrés de la série benzénique et ont l'inconvénient, lorsqu'ils sont incorporés dans des compositions tinctoriales, de conduire à des colorations présentant une ténacité insuffisante, en particulier vis-à-vis des shampooings.

La présente invention vise à proposer de nouvelles compositions pour la coloration d'oxydation des fibres kératiniques et en particulier des fibres kératiniques humaines telles que les cheveux qui permettent d'aboutir à des colorations lumineuses présentant de bonne propriétés de ténacité.

Ainsi, la demanderesse vient en effet de découvrir qu'il est possible d'obtenir de nouvelles teintures à la fois lumineuses et tenaces en associant :

- au moins une base d'oxydation choisie parmi les paraphénylènediamines et les bis-phénylalkylènediamines, et leur sels d'addition avec un acide,
- au moins un coupleur choisi parmi les méta-diphénols, et leurs sels d'addition avec un acide,
- au moins un colorant direct cationique de formule ci-après, et
- au moins un agent oxydant.

L'invention a donc pour premier objet une composition prête à l'emploi pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation choisie parmi les paraphénylènediamines et les bis-phénylalkylènediamines, et leur sels d'addition avec un acide,
- au moins un coupleur choisi parmi les méta-diphénois, et leurs sels d'addition avec un acide,
- au moins un colorant direct cationique:

de formule :

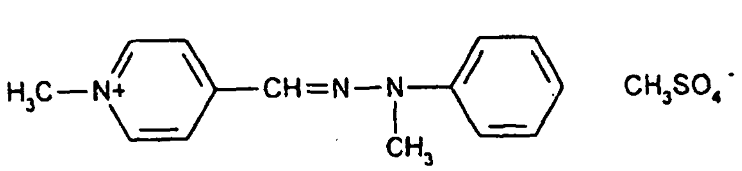

(14)

;

- au moins un agent oxydant.

Les compositions tinctoriales prêtes à l'emploi conformes à l'invention permettent d'aboutir à des colorations dans des nuances naturelles dorées, cendrées ou nacrées présentant une bonne résistance aux différents traitements que peuvent subir les cheveux et en particulier vis-à-vis des shampooings.
L'invention a également pour objet un procédé de teinture d'oxydation des fibres kératiniques mettant en oeuvre cette composition tinctoriale prête à l'emploi.
Les paraphénylènediamines utilisables à titre de base d'oxydation dans les compositions tinctoriales prêtes à l'emploi conformes à l'invention sont de préférence choisies parmi les composés de formule (III) suivante, et leur sels d'addition avec un acide :

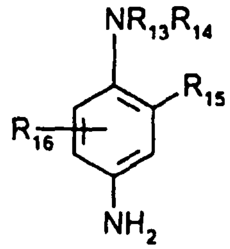

(III)

dans laquelle :

$R_{13}$ représente un atome d'hydrogène, un radical alkyle en $C_1$-$C_4$, monohydroxyalkyle en $C_1$-$C_4$, polyhydroxyalkyle en $C_2$-$C_4$, phényle, 4'-aminophényle ou alcoxy($C_1$-$C_4$)alkyle en $C_1$-$C_4$,
$R_{14}$ représente un atome d'hydrogène, un radical alkyle en $C_1$-$C_4$, monohydroxyalkyle en $C_1$-$C_4$ ou polyhydroxyalkyle en $C_2$-$C_4$,
$R_{15}$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, mésylaminoalcoxy en $C_1$-$C_4$, carbamoylaminoalcoxy en $C_1$-$C_4$ ou acétylaminoalcoxy en $C_1$-$C_4$,
$R_{16}$ représente un atome d'hydrogène ou un radical alkyle en $C_1$-$C_4$.

Parmi les paraphénylènediamines de formule (III) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(βhydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, et leurs sels d'addition avec un acide.
Parmi les paraphénylènediamines de formule (III) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, et leurs sels d'addition avec un acide.
Les bis-phénylalkylènediamines utilisables à titre de base d'oxydation dans les compositions tinctoriales prêtes à l'emploi conformes à l'invention sont de préférence choisies parmi les composés de formule (IV) suivante, et leurs sels d'addition avec un acide :

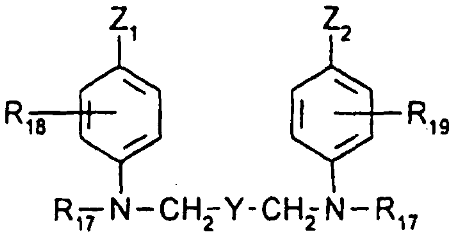

(IV)

dans laquelle :

$Z_1$ et $Z_2$, identiques ou différents, représentent un radical hydroxyle ou $NHR_{20}$ dans lequel $R_{20}$ représente un atome d'hydrogène ou un radical alkyle en $C_1$-$C_4$,
$R_{17}$ représente un atome d'hydrogène, un radical alkyle en $C_1$-$C_4$, monohydroxyalkyle en $C_1$-$C_4$, polyhydroxyalkyle en $C_2$-$C_4$ ou aminoalkyle en $C_1$-$C_4$ dont le reste amino peut être substitué.
$R_{18}$ et $R_{19}$, identiques ou différents, représentent un atome d'hydrogène ou d'halogène ou un radical alkyle en $C_1$-$C_4$,
Y représente un radical pris dans le groupe constitué par les radicaux suivants :

$-(CH_2)_n$ ; $-(CH_2)_m-O-(CH_2)_m$; $-(CH_2)_m-CHOH-(CH_2)_m$ et

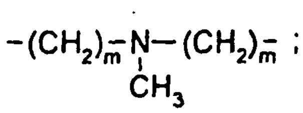

dans lesquels n est un nombre entier compris entre 0 et 8 inclusivement et m est un nombre entier compris entre 0 et 4 inclusivement.

Parmi les bis-phénylalkylènediamines de formule (IV) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.
Parmi ces bis-phénylalkylènediamines de formule (IV), le N,N'-bis-(β-hydroxyéthyl) N,N'-bis-(4'-aminophényl) 1,3-diamino propanol ou l'un de ses sels d'addition avec un acide sont particulièrement préférés.
Les méta-diphénols utilisables à titre de coupleur dans les compositions tinctoriales prêtes à l'emploi conformes à l'invention, sont de préférence choisis parmi les composés de formule (V) suivante, et leurs sels d'addition avec un acide :

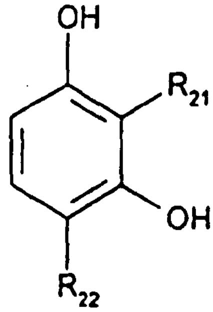

(V)

dans laquelle :

- $R_{21}$ et $R_{22}$, identiques ou différents, représente un atome d'hydrogène, un radical alkyle en $C_1$-$C_4$ ou un atome d'halogène choisi parmi le chlore, le brome ou le fluor.

Parmi les méta-diphénols de formule (V) ci-dessus, on peut plus particulièrement citer le 1,3-dihydroxy benzène, le 2-méthyl 1,3-dihydroxy benzène, le 4-chloro 1,3-dihydroxy benzène, le 2-chloro 1,3-dihydroxybenzène, et leurs sels d'addition avec un acide.

Les sels d'addition avec un acide utilisables dans le cadre des compositions tinctoriales de l'invention sont notamment choisis parmi les chlorhydrates, les carbonates, les bromhydrates, les sulfates et les tartrates.

L'agent oxydant présent dans la composition tinctoriale est choisi parmi les agents oxydants classiquement utilisés en coloration d'oxydation et de préférence parmi le peroxyde d'hydrogène, le peroxyde d'urée, les bromates de métaux alcalins, les persels tels que les perborates et persulfates. Le peroxyde d'hydrogène est particulièrement préféré.

Le ou les colorants directs cationiques de formule conforme à l'invention, représentent de préférence de 0,001 à 10 % en poids environ du poids total de la composition tinctoriale prête à l'emploi et encore plus préférentiellement de 0,05 à 2 % en poids environ de ce poids.

La ou les bases d'oxydation conformes à l'invention, c'est à dire la ou les paraphénylènediamines de formule (III) et/ou la ou les bis-phénylalkylènediamines de formule (IV) représentent de préférence de 0,0001 à 10 % en poids environ du poids total de la composition tinctoriale prête à l'emploi et encore plus préférentiellement de 0,001 à 5 % en poids environ de ce poids.

Le ou les méta-diphénols de formule (V) conformes à l'invention représentent de préférence de 0,0001 à 5 % en poids environ du poids total de la composition tinctoriale prête à l'emploi et encore plus préférentiellement de 0,005 à 3 % en poids environ de ce poids.

Le pH de la composition tinctoriale telle que définie précédemment est généralement compris entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemples, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants, on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (VI) suivante :

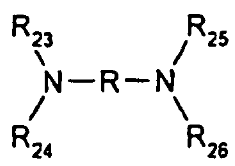

(VI)

dans laquelle R est un reste propylène éventuellement substitué par un groupement hydroxyle ou un radical alkyle en $C_1$-$C_4$ ; $R_{23}$, $R_{24/}$, $R_{25}$ et $R_{26}$, identiques ou différents, représentent un atome d'hydrogène, un radical alkyle en $C_1$-$C_4$ ou hydroxyalkyle en $C_1$-$C_4$.

La composition tinctoriale conforme à l'invention peut encore contenir, en plus des colorants définis ci-dessus, d'autres coupleurs et/ou des colorants directs, notamment pour modifier les nuances ou les enrichir en reflets.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Les compositions tinctoriales prêtes à l'emploi conformes à l'invention peuvent également renfermer divers adjuvants utilisés classiquement dans les compositions pour la teinture des cheveux, tels que des agents tensio-actifs anioniques, cationiques, non-ioniques, amphotères, ou leurs mélanges, des polymères anioniques, cationiques, non-ioniques, amphotères, ou leurs mélanges, des agents épaississants minéraux ou organiques, des agents antioxydants, des agents de pénétration, des agents séquestrant, des parfums, des tampons, des agents dispersants, des agents de conditionnement, des agents filmogènes, des agents conservateurs, des agents opacifiants.

Bien entendu, l'homme de l'art veillera à choisir le ou les éventuels composés complémentaires mentionnés

ci-avant de manière telle que les propriétés avantageuses attachées intrinsèquement à la composition tinctoriale prête à l'emploi conforme à l'invention ne soient pas, ou substantiellement pas, altérées par la ou les adjonctions envisagées.

Les compositions tinctoriales prêtes à l'emploi conformes à l'invention peuvent se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres la composition tinctoriale prête à l'emploi telle que définie précédemment, et on laisse poser pendant 3 à 40 minutes environ, de préférence 5 à 30 minutes environ, après quoi on rince, on lave éventuellement au shampooing, on rince à nouveau et on sèche.

Selon une première forme de réalisation préférée, le procédé comporte une étape préliminaire consistant à stocker sous forme séparée, d'une part, une composition (A) comprenant, dans un milieu approprié pour la teinture, au moins une base d'oxydation choisie parmi les paraphénylènediamines, les bis-phénylalkylènediamines, et leurs sels d'addition avec un acide, au moins un coupleur choisi parmi les méta-diphénols, et leurs sels d'addition avec un acide et au moins un colorant direct cationique de formule telle que définie précédemment et, d'autre part, une composition (B) renfermant, dans un milieu approprié pour la teinture, au moins un agent oxydant tel que défini précédemment, et à procéder à leur mélange au moment de l'emploi avant d'appliquer ce mélange sur les fibres kératiniques.

Selon une deuxième forme de réalisation préférée, le procédé comporte une étape préliminaire consistant à stocker sous forme séparée, d'une part, une composition (A) comprenant, dans un milieu approprié pour la teinture au moins une base d'oxydation choisie parmi les paraphénylènediamines, les bis-phénylalkylènediamines, et leurs sels d'addition avec un acide, au moins un coupleur choisi parmi les méta-diphénols, et leurs sels d'addition avec un acide ; d'autre part une composition (A') comprenant, dans un milieu approprié pour la teinture, au moins un colorant direct cationique de formule telle que définie précédemment ; et enfin, une composition (B) renfermant, dans un milieu approprié pour la teinture, au moins un agent oxydant tel que défini précédemment, et à procéder à leur mélange au moment de l'emploi avant d'appliquer ce mélange sur les fibres kératiniques.

La composition (A') utilisée selon cette deuxième variante du procédé conforme à l'invention, peut éventuellement se présenter sous forme de poudre, le ou les colorants directs cationiques de formule conforme à l'invention constituant alors à lui (eux) seul(s) la totalité de ladite composition (A') ou étant éventuellement dispersé(s) dans un excipient pulvérulent organique et/ou minéral.

Lorsqu'il est présent dans la composition A', l'excipient organique peut être d'origine synthétique ou végétale et est choisi notamment parmi les polymères synthétiques réticulés et non réticulés, les polysaccharides comme les celluloses et les amidons modifiés ou non ainsi que les produits naturels les renfermant tels que la sciure de bois et les gommes végétales (guar, caroube, xanthane, etc...).

Lorsqu'il est présent dans la composition (A'), l'excipient minéral peut être constitué par des oxydes métalliques tels que les oxydes de titane, les oxydes d'aluminium, le kaolin, le talc, les silicates, le mica et les silices. Un excipient avantageusement préféré selon l'invention est la sciure de bois.

La composition (A') en poudre peut encore renfermer des liants ou des produits d'enrobage dans une quantité ne dépassant pas de préférence 3% en poids environ du poids total de ladite composition (A').

Ces liants sont de préférence choisis parmi les huiles et les corps gras liquides d'origine minérale, synthétique, animale ou végétale.

La composition (A) peut éventuellement encore contenir d'autres adjuvants, à l'état de poudre, en particulier des tensio-actifs de toute nature, des agents de conditionnement du cheveu comme par exemple des polymères cationiques, etc...

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus, un second compartiment éventuel renferme la composition (A') telle que définie ci-dessus lorsqu'elle est présente et un troisième compartiment renferme la composition oxydante (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

## Revendications

1. Composition prête à l'emploi pour la teinture d'oxydation des fibres kératiniques et en particulier des fibres kératiniques humaines telles que les cheveux **caractérisée par** la fait qu'elle comprend, dans un milieu approprié pour la teinture :

- au moins une base d'oxydation choisie parmi les paraphénylènediamines et les bis-phénylalkylènediamines, et leur sels d'addition avec un acide.
- au moins un coupleur choisi parmi les méta-diphénols, et leurs sels d'addition avec un acide,
- au moins un colorant direct cationique :

de formule :

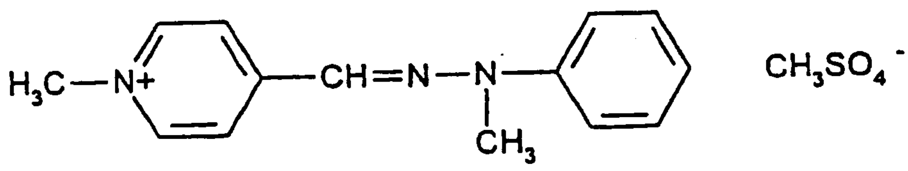

(I4)

;

- au moins un agent oxydant.

**2.** Composition selon la revendication 1, **caractérisée par le fait que** les paraphénylènediamines sont choisies parmi les composés de formule (III) suivante, et leur sels d'addition avec un acide :

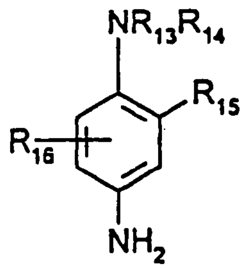

(III)

dans laquelle :

$R_{13}$ représente un atome d'hydrogène, un radical alkyle en $C_1$-$C_4$, monohyoroxyalkyle en $C_1$-$C_4$, polyhydroxyalkyle en $C_2$-$C_4$, phényle, 4'-aminophényle ou alcoxy($C_1$-$C_4$)alkyle en $C_1$-$C_4$,
$R_{14}$ représente un atome d'hydrogène, un radical alkyle en $C_1$-$C_4$, monohydroxyalkyle en $C_1$-$C_4$ ou polyhydroxyalkyle en $C_2$-$C_4$.
$R_{15}$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, mésylaminoalcoxy en $C_1$-$C_4$, carbamoylaminoalcoxy en $C_1$-$C_4$ ou acétylaminoalcoxy en $C_1$-$C_4$,
$R_{16}$ représente un atome d'hydrogène ou un radical alkyle en $C_1$-$C_4$.

**3.** Composition selon la revendication 2, **caractérisée par le fait que** les paraphénylènediamines de formule (III) sont choisies parmi la parachénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2.5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, et leurs sels d'addition avec un acide.

**4.** Composition selon la revendication 1, **caractérisée par le fait que** les bis-phénylalkylènediamines sont choisies parmi les composés de formule (IV) suivante, et leurs sels d'addition avec un acide :

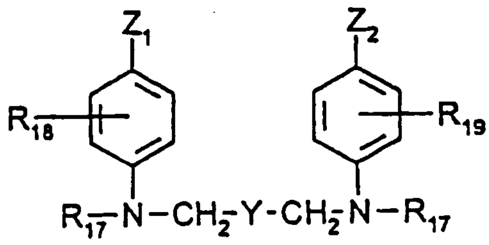

(IV)

dans laquelle :

$Z_1$ et $Z_2$, identiques ou différents, représentent un radical hydroxyle ou $NHR_{20}$ dans lequel $R_{20}$ représente un atome d'hydrogène ou un radical alkyle en $C_1$-$C_4$,
$R_{17}$ représente un atome d'hydrogène, un radical alkyle en $C_1$-$C_4$, monohydroxyalkyle en $C_1$-$C_4$, polyhydroxyalkyle en $C_2$-$C_4$ ou aminoalkyle en $C_1$-$C_4$ dont le reste amino peut être substitué,
$R_{18}$ et $R_{19}$, identiques ou différents, représentent un atome d'hydrogène ou d'halogène ou un radical alkyle en $C_1$-$C_4$,
Y représente un radical pris dans le groupe constitué par les radicaux suivants :

$-(CH_2)_n-$ ; $-(CH_2)_m-O-(CH_2)_m$; $-(CH_2)_m-CHOH-(CH_2)_m$ et

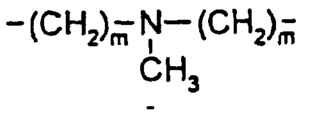 ;

dans lesquels n est un nombre entier compris entre 0 et 8 inclusivement et m est un nombre entier compris entre 0 et 4 inclusivement.

5. Composition selon la revendication 4, **caractérisée par le fait que** les bis-phénylalkylènediamines de formule (IV) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétramethylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

6. Composition selon l'une quelconque des revendications précédentes, **caractérisée par le fait que** les méta-diphénols sont choisis parmi les composés de formule (V) suivante, et leurs sels d'addition avec un acide :

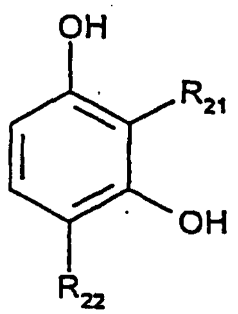

(V)

dans laquelle :

- $R_{21}$ et $R_{22}$, identiques ou différents, représente un atome d'hydrogène, un radical alkyle en $C_1$-$C_4$ ou un atome d'halogène choisi parmi le chlore, le brome ou le fluor.

**7.** Composition selon la revendication 6, **caractérisée par le fait que** les méta-diphénols de formule (V) sont choisis parmi le 1,3-dihydroxy benzène, le 2-méthyl 1,3-dihydroxy benzène, le 4-chloro 1,3-dihydroxy benzène, le 2-chloro 1,3-dihydroxybenzène, et leurs sels d'addition avec un acide.

**8.** Composition selon l'une quelconque des revendications précédentes, **caractérisée par le fait que** les sels d'addition avec un acide sont choisis parmi les chlorhydrates, les carbonates, les bromhydrates, les sulfates et les tartrates.

**9.** Composition selon l'une quelconque des revendications précédentes, **caractérisée par le fait que** l'agent oxydant est choisi parmi le peroxyde d'hydrogène, le peroxyde d'urée, les bromates de métaux alcalins, les persels tels que les perborates et persulfates.

**10.** Composition selon l'une quelconque des revendications précédentes, **caractérisée par** la fait que le colorant direct cationique représente de 0,001 à 10 % en poids du poids total de la composition tinctoriale prête à l'emploi.

**11.** Composition selon rune quelconque des revendications précédentes, **caractérisée par le fait que** la ou les paraphénylènediamines de formule (III) et/ou la ou les bis-phénylalkylènediamines de formule (IV) représentent de 0,0001 à 10 % en poids du poids total de la composition tinctoriale prête à remploi.

**12.** Composition selon l'une quelconque des revendications précédentes, **caractérisée par le fait que** le ou les métadiphértcis de formule (V) représentent de 0,0001 à 5 % en poids du poids total de la composition tinctoriale prête à l'emploi.

**13.** Composition selon l'une quelconque des revendications précédentes, **caractérisée par le fait qu'**elle présente un pH compris entre 5 et 12.

**14.** Composition selon l'une quelconque des revendications précédentes, **caractérisée par le fait que** le milieu approprié pour la teinture est constitué par de l'eau ou par un mélange d'eau et d'au moins un solvant organique.

**15.** Procédé de teinture des fibres kératiniques et en particulier des fibres kératiniques humaines telles que les cheveux **caractérisé par le fait que** l'on applique sur ces fibres au moins une composition tinctoriale telle que définie dans rune quelconque des revendications 1 à 14.

**16.** Procédé selon la revendication 15, **caractérisé par le fait qu'**il comporte une étape préliminaire consistant à stocker sous forme séparée, d'une part, une composition (A) comprenant, dans un milieu approprié pour la teinture, au moins une base d'oxydation choisie parmi les paraphénylènedamines, les bis-phénylalkylènediamines, et leurs sels d'addition avec un acide, au moins un coupleur choisi parmi les méta-diphénols, et leurs sels d'addition avec un acide, et au moins un colorant direct cationique tel que défini dans la revendication 1 et d'autre part une composition (B) renfermant, dans un milieu approprié pour la teinture, au moins un agent oxydant, et à procéder à leur mélange au moment de l'emploi avant d'appliquer ce mélange sur les fibres kératiniques.

**17.** Procédé de teinture selon la revendication 15, **caractérisé par le fait qu'**il comporte une étape préliminaire consistant à stocker sous forme séparée, d'une part, une composition (A) comprenant, dans un milieu approprié pour la teinture au moins une base d'oxydation choisie parmi les paraphénylènediamines, les bis-phénylalkylènediamines, et leurs sels d'addition avec un acide, au moins un coupleur choisi parmi les méta-diphénois, et leurs sels d'addition avec un acide ; d'autre part une composition (A') comprenant, dans un milieu approprié pour la teinture, au moins un colorant direct cationique tel que défini dans la revendication.1 ; et enfin, une composition (B) renfermant, dans un milieu approprié pour la teinture, au moins un agent oxydant et à procéder à leur mélange au moment de l'emploi avant d'appliquer ce mélange sur les fibres kératiniques.

**18.** Procédé selon la revendication 17, **caractérisé par le fait que** la composition (A') se présente sous forme de poudre.

**19.** Dispositif à plusieurs compartiments ou "kit" de teinture, **caractérisé par le fait qu'**un premier compartiment renferme la composition (A) telle que définie à la revendication 16 et un second compartiment renferme une composition oxydante (B).

**20.** Dispositif à plusieurs compartiments ou "kit" de teinture, **caractérisé par le fait qu'**un premier compartiment renferme une composition (A) telle que définie à la revendication 17, un second compartiment referme une composition (A)' telle que définie à la revendication 17 ou 18 et un troisième compartiment renferme une composition oxydante (B).

## Claims

1. Ready-to-use composition for the oxidation dyeing of keratin fibres, and in particular human keratin fibres such as the hair, **characterized in that** it comprises, in a medium which is suitable for dyeing:

   - at least one oxidation base chosen from para-phenylenediamines and bis(phenyl)alkylenediamines, and the addition salts thereof with an acid,
   - at least one coupler chosen from meta-diphenols, and the addition salts thereof with an acid,
   - at least one cationic direct dye of formula:

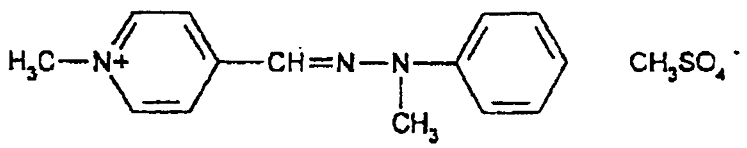

(14)

:

   - at least one oxidizing agent.

2. Composition according to Claim 1, **characterized in that** the para-phenylenediamines are chosen from the compounds of formula (III) below, and the addition salts thereof with an acid:

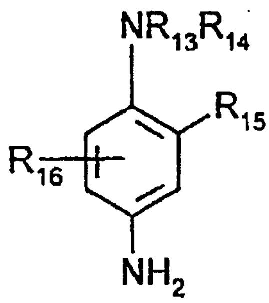

(III)

in which:

$R_{13}$ represents a hydrogen atom, a $C_1$-$C_4$ alkyl, $C_1$-$C_4$ mono-hydroxyalkyl, $C_2$-$C_4$ polyhydroxyalkyl, phenyl, 4'-aminophenyl or ($C_1$-$C_4$)alkoxy($C_1$-$C_4$)alkyl radical,
$R_{14}$ represents a hydrogen atom, a $C_1$-$C_4$ alkyl, $C_1$-$C_4$, monohydroxyalkyl or $C_2$-$C_4$ polyhydroxyalkyl radical,
$R_{15}$ represents a hydrogen atom, a halogen atom such as a chlorine, bromine, iodine or fluorine atom, a $C_1$-$C_4$ alkyl, $C_1$-$C_4$ monohydroxyalkyl, $C_1$-$C_4$ hydroxyalkoxy, $C_1$-$C_4$ mesylaminoalkoxy, $C_1$-$C_4$ carbamoylaminoalkoxy or $C_1$-$C_4$ acetylaminoalkoxy radical,
$R_{16}$ represents a hydrogen atom or a $C_1$-$C_4$ alkyl radical.

3. Composition according to Claim 2, **characterized in that** the para-phenylenediamines of formula (III) are chosen from para-phenylenediamine, para-toluylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-3-methylaniline, 4-amino-3-chloro-N,N-bis(p-hydroxyethyl)aniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β-hydroxyethyl)-para-phenylenediamine, N-(β,γ-dihydroxylpropyl)-para-phenylenediamine, N-(4'-aminophenyl)-para-phenylenediamine, N-phenyl-para-phenylenediamine, 2-β-hydroxyethyloxy-para-phenylenediamine and 2-β-acetylaminoethyloxy-para-phenylenediamine, and the addition salts thereof with an acid.

4. Composition according to Claim 1, **characterized in that** the bis(phenyl)alkylenediamines are chosen from the compounds of formula (IV) below, and the addition salts thereof with an acid:

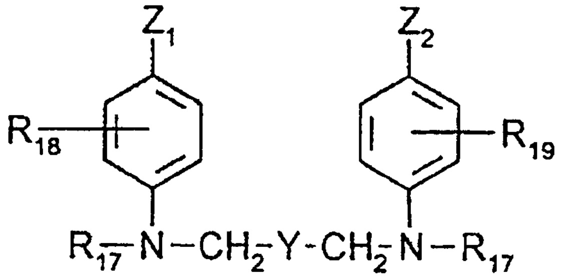

(IV)

in which:

$Z_1$ and $Z_2$, which may be identical or different, represent a hydroxyl radical or $NHR_{20}$ in which $R_{20}$ represents a hydrogen atom or a $C_1$-$C_4$ alkyl radical,
$R_{17}$ represents a hydrogen atom, a $C_1$-$C_4$ alkyl, $C_1$-$C_4$ monohydroxyalkyl or $C_2$-$C_4$ polyhydroxyalkyl radical or a $C_1$-$C_4$ aminoalkyl radical in which the amino residue can be substituted,
$R_{18}$ and $R_{19}$, which may be identical or different, represent a hydrogen or halogen atom or a $C_1$-$C_4$ alkyl radical,
Y represents a radical taken from the group consisting of the following radicals:

$-(CH_2)_n-$ ; $-(CH_2)_m-(CH_2)_m$; $-(CH_2)_m-CHOH-(CH_2)_m$ and

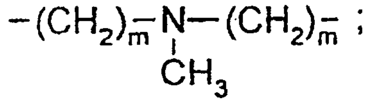

in which n is an integer between 0 and 8 inclusive and m is an integer between 0 and 4 inclusive.

5. Composition according to Claim 4, **characterized in that** the bis(phenyl)alkylenediamines of formula (IV) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine and N,N'-bis(ethyl)-N,N'-bis(4'-amino-3'-methylphenyl) ethylenediamine, and the addition salts thereof with an acid.

6. Composition according to any one of the preceding claims, **characterized in that** the meta-diphenols are chosen from the compounds of formula (V) below, and the addition salts thereof with an acid:

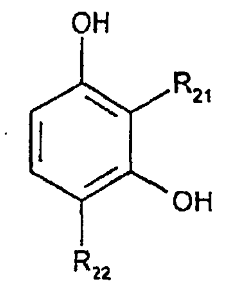

(V)

in which:

- $R_{21}$ and $R_{22}$, which may be identical or different, represent a hydrogen atom, a $C_1$-$C_4$ alkyl radical or a halogen atom chosen from chlorine, bromine and fluorine.

**7.** Composition according to Claim 6, **characterized in that** the meta-diphenols of formula (V) are chosen from 1,3-dihydroxybenzene, 2-methyl-1,3-dihydroxybenzene, 4-chloro-1,3-dirydroxybenzene and 2-chloro-1,3-dihydroxybenzene, and the addition salts thereof with an acid.

**8.** Composition according to any one of the preceding claims, **characterized in that** the addition salts with an acid are chosen from the hydrochlorides, carbonates, hydrobromides, sulfates and tartrates.

**9.** Composition according to any one of the preceding claims, **characterized in that** the oxidizing agent is chosen from hydrogen peroxide, urea peroxide, alkali metal bromates and persalts such as perborates and persulfates.

**10.** Composition according to any one of the preceding claims, **characterized in that** the cationic direct dye represents from 0.001 to 10% by weight relative to the total weight of the ready-to-use dye composition.

**11.** Composition according to any one of the preceding claims, **characterized in that** the para-phenylenediamine(s) of formula (III) and/or the bis(phenyl)alkylenediamine(s) of formula (IV) represent from 0.0001 to 10% by weight relative to the total weight of the ready-to-use dye composition.

**12.** Composition according to any one of the preceding claims, **characterized in that** the metadiphenol(s) of formula (V) represent from 0.0001 to 5% by weight relative to the total weight of the ready-to-use dye composition.

**13.** Composition according to any one of the preceding claims, **characterized in that** it has a pH of between 5 and 12.

**14.** Composition according to any one of the preceding claims, **characterized in that** the medium which is suitable for dyeing consists of water or of a mixture of water and at least one organic solvent.

**15.** Process for dyeing keratin fibres, and in particular human keratin fibres such as the hair, **characterized in that** at least one dye composition as defined in any one of Claims 1 to 14 is applied to these fibres.

**16.** Process according to Claim 15, **characterized in that** it includes a preliminary step which consists in separately storing, on the one hand, a composition (A) comprising, in a medium which is suitable for dyeing, at least one oxidation base chosen from para-phenylenediamines, bis(phenyl)alkylenediamines, and the addition salts thereof with an acid, at least one coupler chosen from meta-diphenols, and the addition salts thereof with an acid, and at least one cationic direct dye as defined in Claim 1, and, on the other hand, a composition (B) containing, in a medium which is suitable for dyeing, at least one oxidizing agent, and in mixing them together at the time of use before applying this mixture to the keratin fibres.

**17.** Dyeing process according to Claim 15, **characterized in that** it includes a preliminary step which consists in separately storing, on the one hand, a composition (A) comprising, in a medium which is suitable for dyeing, at least one oxidation base chosen from para-phenylenediamines, bis(phenyl)alkylenediamines, and the addition salts thereof with an acid, at least one coupler chosen from meta-diphenols, and the addition salts thereof with an acid; on the other hand, a composition (A') comprising, in a medium which is suitable for dyeing, at least one cationic direct dye as defined in Claim 1; and, lastly, a composition (B) containing, in a medium which is suitable for dyeing, at least one oxidizing agent, and in mixing them together at the time of use before applying this mixture to the keratin fibres.

**18.** Process according to Claim 17, **characterized in that** the composition (A') is in powder form.

**19.** Multi-compartment dyeing "kit" or device, **characterized in that** a first compartment contains the composition (A) as defined in Claim 16 and a second compartment contains an oxidizing composition (B).

**20.** Multi-compartment dyeing "kit" or device, **characterized in that** a first compartment contains a composition (A) as defined in Claim 17, a second compartment contains a composition (A') as defined in Claim 17 or 18, and a third compartment contains an oxidizing composition (B).

## Patentansprüche

**1.** Gebrauchsfertige Zusammensetzung zum oxidativen Färben von Keratinfasern und insbesondere menschlichen Keratinfasern, wie Haaren, **dadurch gekennzeichnet, dass** sie in einem zum Färben geeigneten Medium enthält:

- mindestens eine Oxidationsbase, die unter den p-Phenylendiaminen, den Bisphenylalkylendiaminen und den Additionssalzen dieser Verbindungen mit einer Säure ausgewählt ist,
- mindestens einen Kuppler, der unter den m-Diphenolen und den Additionssalzen dieser Verbindungen mit einer Säure ausgewählt ist,
- mindestens einen kationischen Direktfarbstoff der folgenden Formel:

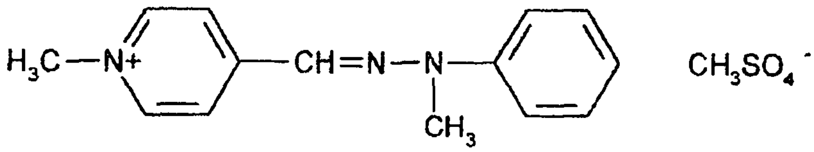

(I4)

;

und
- mindestens ein Oxidationsmittel.

**2.** Zusammensetzung nach Anspruch 1, **dadurch gekennzeichnet, dass** die p-Phenylendiamine unter den Verbindungen der folgenden Formel (III) und den Additionssalzen dieser Verbindungen mit einer Säure ausgewählt sind:

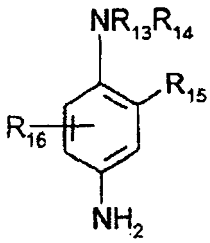

(III)

worin bedeuten:

$R_{13}$ ein Wasserstoffatom, $C_{1-4}$-Alkyl, $C_{1-4}$-Monohydroxyalkyl, $C_{2-4}$-Polyhydroxyalkyl, Phenyl, 4'-Aminophenyl oder $C_{1-4}$-Alkoxy-$C_{1-4}$-alkyl,
$R_{14}$ ein Wasserstoffatom, $C_{1-4}$-Alkyl, $C_{1-4}$-Monohydroxyalkyl oder $C_{2-4}$-Polyhydroxyalkyl,
$R_{15}$ ein Wasserstoffatom, ein Halogenatom, wie Chlor, Brom, Iod oder Fluor, $C_{1-4}$-Alkyl, $C_{1-4}$-Monohydroxyalkyl, $C_{1-4}$-Hydroxyalkoxy, $C_{1-4}$-Mesylaminoalkoxy, $C_{1-4}$-Carbamoyl-aminoalkoxy oder $C_{1-4}$-Acetylaminoalkoxy,
$R_{16}$ ein Wasserstoffatom oder eine $C_{1-4}$-Alkylgruppe.

**3.** Zusammensetzung nach Anspruch 2, **dadurch gekennzeichnet, dass** die p-Phenylendiamine der Formel (III) ausgewählt sind unter: p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin,4-Amino-N,N-diethyl-3-methylanilin, N,N-Bis-(β-hydroxyethyl)-p-phenylendiamin, 4-Amino-N,N-bis-(β-hydroxyethyl)-3-methylanilin, 4-Amino-3-chlor-N,N-bis-(β-hydroxyethyl)-anilin, 2-β-Hydroxyethyl-p-phenylendiamin,2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylamino-ethyloxy-p-phenylendiamin und den Additionssalzen dieser Verbindungen mit einer Säure.

**4.** Zusammensetzung nach Anspruch 1, **dadurch gekennzeichnet, dass** die Bisphenylalkylendiamine unter den Verbindungen der folgenden Formel (IV) und den Additionssalzen dieser Verbindungen mit einer Säure ausgewählt sind:

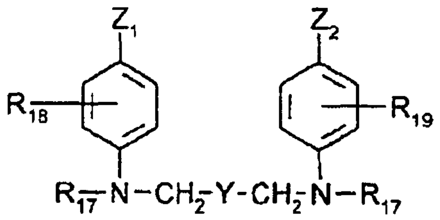 (IV)

worin bedeuten:

$Z_1$ und $Z_2$, die identisch oder voneinander verschieden sind, eine Hydroxygruppe oder $NHR_{20}$, wobei $R_{20}$ Wasserstoff oder $C_{1-4}$-Alkyl bedeutet,
$R_{17}$ ein Wasserstoffatom, $C_{1-4}$-Alkyl, $C_{1-4}$-Monohydroxyalkyl, $C_{2-4}$-Polyhydroxyalkyl oder $C_{1-4}$-Aminoalkyl, wobei die Aminogruppe substituiert sein kann,
$R_{18}$ und $R_{19}$, die identisch oder voneinander verschieden sind, Wasserstoff oder Halogen oder eine $C_{1-4}$-Alkylgruppe,
Y eine Gruppe, die unter den folgenden Gruppen ausgewählt ist:

$-(CH_2)_n-$ ; $-(CH_2)_m-O-(CH_2)_m$; $-(CH_2)_m-CHOH-(CH_2)_m$ und

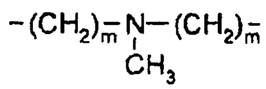 ;

worin n 0 oder eine ganze Zahl von 1 bis 8 und m 0 oder eine ganze Zahl von 1 bis 4 bedeuten.

5. Zusammensetzung nach Anspruch 4, **dadurch gekennzeichnet, dass** die Bisphenylalkylendiamine der Formel (TV) ausgewählt sind unter: N,N'-Bis-(β-hydroxyethyl)-N,N'-bis-(4'-aminophenyl)-1,3-diaminopropanol, N,N'-Bis-(β-hydroxyethyl)-N,N'-bis-(4'-aminophenyl)-ethylendiamin, N,N'-Bis-(4-aminophenyl)-tetraethylendiamin, N-N'-Bis-(β-hydroxyethyl)-N,N'-bis-(4-aminophenyl)-tetramethylendiamin, N,N'-Bis-(4-methylaminophenyl)-tetramethylendiamin, N,N'-Bis-(ethyl)-N,N'-bis-(4'-arnino-3'-methylphenyl)-ethylendiamin und den Additionssalzen dieser Verbindungen mit einer Säure.

6. Zusammensetzung nach einem der vorhergehenden Ansprüche, **dadurch gekennzeichnet, dass** die m-Diphenole unter den Verbindungen der folgenden Formel (V) und den Additionssalzen dieser Verbindungen mit einer Säure ausgewählt ist:

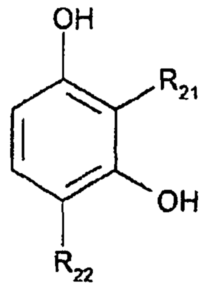 (V)

worin bedeuten:

- $R_{21}$ und $R_{22}$, die identisch oder voneinander verschieden sind, ein Wasserstoffatom, eine $C_{1-4}$-Alkylgruppe oder ein Halogenatom, das unter Chlor, Brom oder Fluor ausgewählt ist.

7. Zusammensetzung nach Anspruch 6, **dadurch gekennzeichnet, dass** die m-Diphenole der Formel (V) ausgewählt sind unter: 1,3-Dihydroxybenzol, 2-Methyl-1,3-dihydroxybenzol, 4-Chlor-1,3-dihydroxybenzol, 2-Chlor-1,3-dihydroxybenzol und den Additionssalzen dieser Verbindungen mit einer Säure.

**8.** Zusammensetzung nach einem der vorhergehenden Ansprüche, **dadurch gekennzeichnet, dass** die Additionssalze mit einer Säure unter den Hydrochloriden, Carbonaten, Hydrobromiden, Sulfaten und Tartraten ausgewählt sind.

**9.** Zusammensetzung nach einem der vorhergehenden Ansprüche, **dadurch gekennzeichnet, dass** das Oxidationsmittel unter Wasserstoffperoxid, Harnstoffperoxid, Alkalimetallbromaten und Salzen von Persäuren, wie Perboraten und Persulfaten, ausgewählt ist.

**10.** Zusammensetzung nach einem der vorhergehenden Ansprüche, **dadurch gekennzeichnet, dass** der kationische Direktfarbstoff 0,001 bis 10 Gew.-% des Gesamtgewichts der gebrauchsfertigen Färbemittelzusammensetzung ausmachen.

**11.** Zusammensetzung nach einem der vorhergehenden Ansprüche, **dadurch gekennzeichnet, dass** das p-Phenylendiamin oder die p-Phenylendiamine der Formel (III) und/oder das Bisphenylalkylendiamin oder die Bisphenylalkylendiamine der Formel (IV) 0,0001 bis 10 Gew.-% des Gesamtgewichts der gebrauchsfertigen Färbemittelzusammensetzung ausmachen.

**12.** Zusammensetzung nach einem der vorhergehenden Ansprüche, **dadurch gekennzeichnet, dass** das m-Diphenol oder die m-Diphenole der Formel (V) 0,0001 bis 5 Gew.-% des Gesamtgewichts der gebrauchsfertigen Färbemittelzusammensetzung ausmachen.

**13.** Zusammensetzung nach einem der vorhergehenden Ansprüche, **dadurch gekennzeichnet, dass** sie einem pH-Wert im Bereich von 5 bis 12 aufweist.

**14.** Zusammensetzung nach einem der vorhergehenden Ansprüche, **dadurch gekennzeichnet, dass** das zum Färben geeignete Medium aus Wasser oder einem Gemisch aus Wasser und mindestens einem organischen Lösungsmittel besteht.

**15.** Verfahren zum Färben von Keratinfasern und insbesondere von menschlichen Keratinfasern, wie Haaren, **dadurch gekennzeichnet, dass** auf die Fasern mindestens eine Färbemittelzusammensetzung nach einem der Ansprüche 1 bis 14 aufgetragen wird.

**16.** Verfahren nach Anspruch 15, **dadurch gekennzeichnet, dass** es einen vorbereitenden Schritt umfasst, der darin besteht, getrennt voneinander einerseits eine Zusammensetzung (A), die in einem zum Färben geeigneten Medium mindestens eine Oxidationsbase, die unter den p-Phenylendiaminen, Bisphenylaklylendiaminen und den Additionssalzen dieser Verbindungen mit einer Säure ausgewählt ist, mindestens einen Kuppler, der unter den m-Diphenolen und den Additionssalzen dieser Verbindungen mit einer Säure ausgewählt ist, und mindestens einen wie in Anspruch 1 definierten kationischen Direktfarbstoff enthält, und andererseits eine Zusammensetzung (B) aufzubewahren, die in einem zum Färben geeigneten Medium mindestens ein Oxidationsmittel enthält, und diese bei der Anwendung zu vermischen, bevor das Gemisch auf die Keratinfasern aufgetragen wird.

**17.** Verfahren zum Färben nach Anspruch 15, **dadurch gekennzeichnet, dass** es einen vorbereitenden Schritt umfasst, der darin besteht, einerseits eine Zusammensetzung (A), die in einem zum Färben geeigneten Medium mindestens eine Oxidationsbase, die unter den p-Phenylendiaminen, Bisphenylalkylendiaminen und den Additionssalzen dieser Verbindungen mit einer Säure ausgewählt ist, und mindestens einen Kuppler enthält, der unter den m-Diphenolen und den Additionssalzen dieser Verbindungen mit einer Säure ausgewählt ist, und andererseits eine Zusammensetzung (A'), die in einem zum Färben geeigneten Medium mindestens einen wie in Anspruch 1 definierten kationischen Direktfarbstoff enthält, und schließlich eine Zusammensetzung (B) getrennt voneinander aufzubewahren, die in einem zum Färben geeigneten Medium mindestens ein Oxidationsmittel enthält, und diese bei der Anwendung zu vermischen, bevor das Gemisch auf die Keratinfasern aufgebracht wird.

**18.** Verfahren nach Anspruch 17, **dadurch gekennzeichnet, dass** die Zusammensetzung (A') in Pulverform vorliegt.

**19.** Vorrichtung mit mehreren Abteilungen oder "Kit" zum Färben, **dadurch gekennzeichnet, dass** eine erste Abteilung die in Anspruch 16 definierte Zusammensetzung (A) und eine zweite Abteilung die oxidierende Zusammensetzung (B) enthält.

**20.** Vorrichtung mit mehreren Abteilungen oder "Kit" zum Färben, **dadurch gekennzeichnet, dass** eine erste Abteilung

eine in Anspruch 17 definierte Zusammensetzung (A), eine zweite Abteilung eine in Anspruch 17 oder 18 definierte Zusammensetzung (A') und eine dritte Abteilung eine oxidierende Zusammensetzung (B) enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2586913